(19) Europäisches Patentamt
European Patent Office
Office européen des brevets

(11) Veröffentlichungsnummer: **0 011 091**
**B1**

# (12) EUROPÄISCHE PATENTSCHRIFT

(45) Veröffentlichungstag der Patentschrift: **15.07.81**

(51) Int. Cl.³: **C 07 C 148/00**, C 07 C 149/18, C 07 C 149/36

(21) Anmeldenummer: **79102965.5**

(22) Anmeldetag: **16.08.79**

## (54) Verfahren zur Herstellung von Alkyl- bzw. Arylthiomethyl-phenolen.

(30) Priorität: **01.09.78 DE 2838273**

(43) Veröffentlichungstag der Anmeldung: **28.05.80 Patentblatt 80/11**

(45) Bekanntmachung des Hinweises auf die Patenterteilung: **15.07.81 Patentblatt 81/28**

(84) Benannte Vertragsstaaten: **BE CH DE FR GB IT NL**

(56) Entgegenhaltungen:
**DE-C3-1 910 588**

**THE JOURNAL OF THE AMERICAN CHEMICAL SOCIETY, Band 71, 1949, Easton I. W. RUDERMAN et al.: «A Condensation Reaction of Thiols with Phenol Alcohols»**

(73) Patentinhaber: **BAYER AG, Zentralbereich Patente, Marken und Lizenzen, D-5090 Leverkusen 1, Bayerwerk (DE)**

(72) Erfinder: **Wedemeyer, Karlfried, Dr., Bilharzstrasse 7, D-5000 Köln (DE)**
Erfinder: **Fiege, Helmut, Dr., Walter-Flex-Strasse 8, D-5090 Leverkusen-1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 011 091 B1

# Verfahren zur Herstellung von Alkyl- bzw. Arylthiomethylphenolen

Die vorliegende Erfindung betrifft ein neues Verfahren zur Herstellung von Alkyl- bzw. Arylthiomethylphenolen. Diese dienen als Zwischenprodukte zur Herstellung von Pflanzenschutzmitteln.

Es ist bekannt, dass man z.B. Äthylthiomethylphenol durch Umsetzen von 2-Chlormethylphenol mit Äthylmercaptan bei 0–10° in Acetonitril als Lösungsmittel erhalten kann. Die Ausbeute beträgt jedoch nur 50% d. Theorie, und es sind äquimolare Mengen Natriummethylat als Hilfsbase erforderlich (DT-PS 1 910 588). Ferner ist das Ausgangsmaterial 2-Chlormethylphenol technisch nur schwer zugänglich, zum einen, weil seine Herstellung durch Chlormethylierung von Phenol unbefriedigend verläuft, zum anderen, weil bei seiner Herstellung – wie bei allen Chlormethylierungen – mit der Bildung von Chlormethyläthern zu rechnen ist, die als stark cancerogen bekannt sind [s. Houben-Weyl, Bd. 6/1c, S. 1042, 4. Aufl. (1976)].

Um diese Schwierigkeiten zu umgehen, geht man von Methylphenolen aus, deren OH-Gruppe zunächst durch Verestern geschützt wird. Dann wird die Methylgruppe des so geschützten Phenols durch Chlorieren in die Chlormethylgruppe übergeführt, diese dann mit Alkalimercaptid zur Alkylthiomethylgruppe umgesetzt und danach das Alkylthiomethylphenol durch alkalische Verseifung der Estergruppe freigesetzt [JA 74 20 191; C.A. 82, 86 197x (1975)]. Das Verfahren beinhaltet also vier Reaktionsschritte, die verlustreich und technisch wenig attraktiv sind.

Es ist ferner bekannt, dass man 4-Methyl-2,6-bis-butylthiomethylphenol durch Umsetzen von (1 Mol) 4-Methyl-2,6-bis-hydroxymethylphenol mit (10 Mol) N-Butylmercaptan in (25 Mol) Eisessig erhält. Als Voraussetzung für den Ablauf dieser Reaktion werden Wasserfreiheit der Einsatzprodukte und die Anwesenheit von wasserfreiem Chlorwasserstoff oder Bortrifluorid genannt (I.W. Ruderman, E.M. Fettes, J. Am. Chem. Soc. 71 (1949), 2264).

Abgesehen von der mässigen Ausbeute (60% d.Th.), der langen Reaktionszeit (ca. 2 Tage) und den technisch wenig vorteilhaften Bedingungen (Wasserfreiheit der Einsatzmaterialien, Anwendung von HCl oder $BF_3$, die in Verbindung mit dem Reaktionswasser zu einem sehr korrosiven Reaktionsgemisch führen, schlechte Raum/Zeit-Ausbeuten, aufwendige Regenerierung und Entwässerung der im sehr grossen Überschuss eingesetzten Materialien Essigsäure und Mercaptan) besteht ein wesentlicher Nachteil des Verfahrens in der Einschränkung auf Hydroxymethylphenole mit nicht mehr reaktionsfähigen (substituierten) o- und/oder p-Stellen zur phenolischen OH-Gruppe.

Es ist weiterhin bekannt, dass man Aryl- oder Alkylthiomethylnaphthole in einer Mannichreaktion durch Umsetzung z.B. von β-Naphthol mit Formaldehyd und Aryl- oder Alkylmercaptan erhält (vgl. F. Poppelsdorf, S. J. Holt, J. Chem. Soc. 1954, 1124 ff.). Die Reaktion läuft jedoch nur ab, wenn gleichzeitig Triäthylamin als Hilfsbase anwesend ist, und auch dann erfordert sie zur Erzielung befriedigender Ausbeuten Reaktionszeiten von ca. 6 Tagen.

Überträgt man diese Reaktion auf Phenol, so entsteht kein einheitliches Reaktionsprodukt, sondern ein Gemisch von Isomeren, Homologen und Harzen (vgl. US-PS 2 322 376, 1943).

Es ist ebenfalls bekannt, dass man Alkylthiomethyl-phenolgemische durch Umsetzen von Dialkylaminomethylphenolen mit Alkylmercaptanen erhält (vgl. US-PS 2 417 118, 1947). In diesem Fall dient offensichtlich das Dialkylaminophenol bzw. das bei der Reaktion als Coprodukt entstehende Dialkylamin als Hilfsbase.

Dieses Verfahren besitzt ebenfalls erhebliche technische Nachteile: Z.B. entstehen bei diesem Verfahren Gemische isomerer und homologer Alkylthiomethylphenole. Ferner betragen die Reaktionszeiten 35–60 Stunden. Dies hat zusätzliche Ausbeuteverluste durch Verharzung zur Folge, da die als Ausgangsverbindungen dienenden Dialkylaminomethylphenole bei den erforderlichen Reaktionstemperaturen thermisch nicht stabil sind und während der langen Reaktionszeit zum Teil verharzen.

Es ist ferner bekannt, dass man Alkyl- bzw. Arylthiomethylphenole durch Umsetzen von Dialkylaminomethylphenolen mit Thiocarbonsäure-S-alkyl- bzw.-arylestern erhalten kann (vgl. DE-AS 2 614 875, 1976).

Dieses Verfahren hat den Nachteil, dass die in die Reaktion einzusetzenden Thiocarbonsäure-S-ester im Vergleich zum Mercaptan relativ wertvolle Reaktionskomponenten sind, die erst in einem vorausgehenden Reaktionsschnitt aus dem Mercaptan und einem Carbonsäurehalogenid hergestellt werden müssen. Ein weiterer Nachteil des Verfahrens ist, dass die Dialkylamino-Komponente des eingesetzten Dialkylaminomethylphenols als Carbonsäuredialkylamid verloren geht. Letzteres fällt bei dem Verfahren als Coprodukt in verunreinigter Form an und muss verbrannt oder kostspielig aufgearbeitet werden. Das Verfahren ist also mit der Vergeudung wertvoller Chemikalien bzw. hohen Kosten verbunden.

Es ist somit noch kein Verfahren bekannt, das es in befriedigender Weise gestattet, Alkyl- bzw. Arylthiomethylphenole ohne beträchtlichen technischen Aufwand und Aufwand an Hilfschemikalien herzustellen.

Es wurde nun gefunden, dass man in hoher Ausbeute und Reinheit Alkyl- bzw. Arylthiomethylphenole der Formel

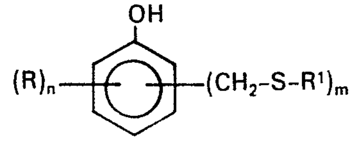

I

in der
m für 1, 2 oder 3 steht,
n für die Zahl steht, die sich aus der Differenz (5-m) ergibt, und
R entweder einzeln und unabhängig voneinander für Wasserstoff, Alkyl, Cycloalkyl, Aryl, Aralkyl, Alkoxy, Cycloalkoxy, Aryloxy, Halogen, Nitro oder für einen an den Phenylring ankondensierten Benzolring oder Cycloalkanring steht und
$R^1$ für gegebenenfalls substituiertes Alkyl mit 1-12 C-Atomen oder für gegebenenfalls substituiertes Phenyl oder gegebenenfalls substituiertes Aralkyl steht,
erhält, wenn man Hydroxymethylphenole der Formel

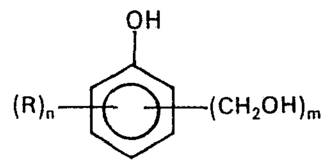

II

mit Mercaptanen oder Thiophenolen der Formel

$R^1$-S-H III

bei Temperaturen von 20 bis 200°, gegebenenfalls in Anwesenheit eines Verdünnungsmittels umsetzt.

Es ist als ausgesprochen überraschend zu bezeichnen, dass bei dem erfindungsgemässen Verfahren Alkyl- bzw. Arylthiomethylphenole in guten Ausbeuten gebildet werden: Nach dem Stand der Technik war – insbesondere bei Hydroxymethylphenolen mit freien ortho- und/oder para-Positionen – unter den erfindungsgemässen Bedingungen mit einer weitgehenden Verharzung zu rechnen, denn bekanntlich treten Hydroxymethylphenole ja als thermisch und chemisch äusserst labile Zwischenstufen bei der Phenol-Formaldehyd-Harz-Fabrikation auf (vgl. H. Wagner u. H.F. Sarx, Lackkunstharze, Carl Hanser Verlag, München 1971, S. 35-36). Ferner war zu erwarten, dass die Umsetzung des Mercaptans mit der Hydroxymethylgruppe den Zusatz von stark sauren oder stark basischen Hilfsstoffen (Katalysatoren) erfordert, die wiederum die Verharzungstendenz fördern würden.

Das erfindungsgemässe Verfahren weist eine Reihe von Vorteilen auf: So entfällt die Verwendung von Hilfsstoffen und Katalysatoren. Als Co-Produkt entsteht nur Wasser, das leicht, z.B. destillaltiv, abzutrennen ist. Die Mercaptane können direkt in die Reaktion eingesetzt werden und brauchen nicht erst in eine andere Verbindung übergeführt zu werden. Die Einsatzmaterialien brauchen nicht wasserfrei zu sein. Es treten keine besonderen Korrosionsprobleme auf. Durch die Wahl einer entsprechend hohen Temperatur können kurze Reaktionszeiten und damit hohe Raum/Zeit-Ausbeuten erzielt werden. Durch Anwendung eines entsprechenden Verdünnungsmittels kann auch drucklos gearbeitet werden. Die Reaktionsparameter sind in weiten Grenzen frei wählbar und damit gut den technischen Verhältnissen anzupassen.

Bei Verwendung von 2-Hydroxymethyl-phenol und Äthylmercaptan als Ausgangsverbindungen kann der Reaktionsablauf durch das folgende Formelschema wiedergegeben werden:

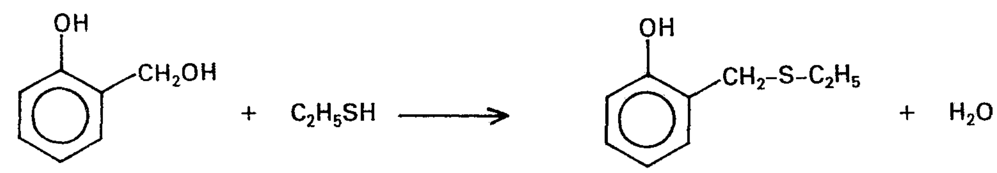

Die erfindungsgemäss als Ausgangsstoffe zu verwendenden Hydroxymethylphenole sind durch die Formel (II) allgemein definiert. In dieser Formel stehen die Reste R einzeln und unabhängig voneinander vorzugsweise für Wasserstoff, Alkyl mit 1 bis 6 C-Atomen, Cycloalkyl mit 3 bis 6 C-Atomen, Phenyl, Benzyl oder Halogen, insbesondere Chlor.

Hydroxymethylphenole der Formel (II) sind an sich bekannt oder können nach bekannten Verfahren hergestellt werden, beispielsweise durch Addition von Formaldehyd an Phenole (vgl. J.F. Walker, «Formaldehyde», Reinhold, New York, 3. Aufl., 1964, S. 310 ff.) oder durch Reduktion von Phenolaldehyden.

Die erfindungsgemäss als Ausgangsstoffe zu verwendenden Mercaptane sind durch Formel (III) allgemein definiert. In dieser Formel steht der Rest $R^1$ vorzugsweise für Alkyl mit 1 bis 12 C-Atomen oder alkoxysubstituiertes Alkyl, Cycloalkyl mit 3 bis 6 C-Atomen, Phenyl, Benzyl, alkyl- und/oder halogensubstituiertes Phenyl.

Mercaptane der Formel (III) sind an sich bekannt oder können analog bekannter Verfahren hergestellt werden.

Bei der Durchführung des erfindungsgemässen Verfahrens kann die auf 1 Mol umzusetzende Hydroxymethylgruppe im Hydroxymethylphenol einzusetzende Mercaptanmenge in weiten Grenzen schwanken. Gewöhnlich reichen Mengen um 1 Mol Mercaptan pro Mol Hydroxymethylgruppe aus. Da das Mercaptan gleichzeitig auch als Verdünnungsmittel dienen kann, sind dem Mercaptan/Hydroxymethylphenol-Verhältnis nach oben keine Grenzen gesetzt.

Bei dem erfindungsgemässen Verfahren kann als Verdünnungsmittel ausser dem betreffenden Mercaptan selbst jedes beliebige inerte protische oder aprotische Lösungsmittel verwendet werden.

Als Verdünnungsmittel werden vorzugsweise Wasser, aliphatische, cycloaliphatische und aromatische Kohlenwasserstoffe (die auch chloriert sein können) wie z.B. Benzine, Petroläther, Toluol, Xylol, Chlorbenzol, Cyclohexan, Methylcyclohexan, Tetralin, Decalin, Alkohole wie z.B. Methanol, Äthanol, Isopropanol, Butanol, Cyclohexanol, Methylcyclohexanol, Äthylenglycol, Äther wie z.B. Diäthyläther, Diisopropyläther, Di-n-butyläther, Dioxan, Tetrahydrofuran, Diglym, Ätheralkohole wie z.B. Methylglycol, Äthylglycol, Butylglycol, Methyldiglycol, Äthyldiglycol, Phenole wie z.B. Phenol, Kresol, Xylenol bzw. das dem Hydroxymethylphenol zu Grunde liegende Phenol, Ketone wie z.B. Aceton, Methyläthylketon, Methylisobutylketon, Cyclohexanon, Carbonsäurederivate wie z.B. Dimethylformamid, Dimethylacetamid, N-Methylpyrrolidon, Nitrile wie z.B. Acetonitril oder Propionitril, sonstige stickstoffhaltige organische Lösungsmittel wie z.B. Pyridin, Picolin, Lutidin, schwefelhaltige organische Verbindungen wie z.B. Dimethylsulfoxid oder das erfindungsgemäss entstehende Reaktionsgemisch, verwendet werden. Auch Gemische dieser Verdünnungsmittel können verwendet werden.

Die Verwendung eines Verdünnungsmittels ist nicht zwingend vorgeschrieben. Im wesentlichen verwendet man es aus reaktionstechnischen Gründen, beispielsweise, um eine bessere Durchmischung, einen besseren Wärmeaustausch und eine sichere Reaktionsführung zu gewährleisten. Demgemäss kann das Verhältnis von Verdünnungsmittel zu Hydroxymethylphenol in weiten Grenzen frei gewählt werden. Bevorzugt sind Gewichtsverhältnisse von Verdünnungsmittel zu Hydroxymethylphenol von 1:1 bis 20:1. Es ist nicht erforderlich, dass sich alle Einsatzprodukte und/oder Reaktionsprodukte vollständig in dem Verdünnungsmittel lösen, d.h. das Einsatz- und/oder das Reaktionsgemisch können auch mehrphasig sein. Dies kann sogar vorteilhaft für die Aufarbeitung des Reaktionsproduktes sein. Beispielsweise fällt bei der Umsetzung in Wasser als Verdünnungsmittel ein zweiphasiges Reaktionsgemisch an, das durch einfache Phasentrennung in das Alkyl- bzw. Arylthiomethylphenol und eine wässrige Phase zerlegt werden kann. Letztere kann (nach Abnahme einer dem Reaktionswasser entsprechenden Menge) direkt in den nächsten Ansatz zurückgeführt werden; es braucht also nicht destillativ entfernt zu werden.

Das erfindungsgemässe Verfahren kann innerhalb eines weiten Temperaturbereichs durchgeführt werden, im allgemeinen wird die Umsetzung bei Temperaturen zwischen 20 und 200°, vorzugsweise zwischen 50 und 180°, durchgeführt.

Die Umsetzung kann bei Normaldruck durchgeführt werden, beispielsweise auch unter Rückfluss, oder unter Eigendruck im Autoklaven. Die Arbeitsweise richtet sich nach dem Dampfdruck des Reaktionsgemisches, den technischen Gegebenheiten und der gewünschten Reaktionszeit. Das Arbeiten unter Eigendruck im Autoklaven ermöglicht höhere Reaktionstemperaturen und damit kürzere Reaktionszeiten. Die Arbeitsweise im Autoklaven kann bei niedrigsiedenden Mercaptanen und niedrigsiedenden Lösungs- bzw. Verdünnungsmitteln zweckmässig sein. Hochsiedende Lösungs- und Verdünnungsmittel ermöglichen ein Arbeiten bei höheren Temperaturen auch unter Normaldruck.

Für das erfindungsgemässe Verfahren ist es nicht erforderlich, dass die Hydroxymethylphenole (II) vor ihrer erfindungsgemässen Umsetzung mit dem Mercaptan (III) in isolierter (freier) Form vorliegen. Es ist ein grosser Vorteil des Verfahrens, dass man das Hydroxymethylphenol nicht erst in freier Form isolieren muss, sondern seine Umsetzung mit dem Mercaptan gegebenenfalls bereits in dem Medium vornehmen kann, in dem das Hydroxymethylphenol bei seiner Herstellung oder Isolierung anfällt. Beispielsweise kann man das Hydroxymethylphenol durch Umsetzen von Formaldehyd in überschüssigem Phenol herstellen und dann zu diesem Reaktionsgemisch das Mercaptan geben, um die erfindungsgemässe Umsetzung zum Thiomethylphenol (I) vorzunehmen. Als Verdünnungsmittel dient in diesem Fall das dem Hydroxymethylphenol zu Grunde liegende, im Überschuss zugegebene Phenol. Dadurch entfällt die Isolierung des Hydroxymethylphenols, und die Reaktion läuft praktisch auf eine Eintopf-Reaktion zwischen Formaldehyd, dem Phenol und dem Mercaptan hinaus.

Der gleiche Fall liegt vor, wenn man z.B. einen geeigneten Phenolaldehyd in einem Alkohol löst, den Phenolaldehyd (mit Raney-Nickel) zum Hydroxymethylphenol reduziert und dann (ggf. nach Abtrennen des Kontaktes) zum Reaktionsgemisch das Mercaptan gibt und die erfindungsgemässe Umsetzung zum Thiomethylphenol (II) vornimmt.

Die Isolierung des entstandenen Alkyl- bzw. Arylthiomethylphenols erfolgt durch Abtrennen von den Verdünnungsmitteln nach bekannten Methoden. Das Verdünnungsmittel kann bei der erfindungsgemässen Umsetzung wiederverwendet werden. Nebenprodukte sind gegebenenfalls ganz oder teilweise nach gängigen Verfahren zu entfernen. Das als Nebenprodukt anfallende Wasser kann auch schon während der Reaktion entfernt werden.

Die nach dem erfindungsgemässen Verfahren herstellbaren Alkyl- bzw. Arylthiomethylphenole können als Zwischenprodukte zur Synthese von Pflanzenschutzmitteln, insbesondere insektiziden Wirkstoffen wie z.B. 2-(Äthylthiomethylphenyl)-N-methyl-carbamat, verwendet werden (vgl. DT-PS 1 910 588, DE-OS 2 122 311).

Beispiel 1

Eine Mischung von 99,2 g (0,8 Mol) 2-Hydroxymethyl-phenol und 124 g (2 Mol) Äthylmercaptan wird unter Rühren 5 Stunden auf 170 °C in einem VA-Autoklaven erhitzt. Nach Abkühlen auf Raumtemperatur werden das nicht umgesetzte Äthylmercaptan und das Reaktionswasser bei Normaldruck und anschliessend das 2-Äthylthiomethyl-phenol bei $1{,}999 \cdot 10^{-3}$ Bar (1,5 Torr) abdestilliert. Erhalten werden 128 g 2-Äthylthiomethyl-phenol ≙95% der Theorie.

Beispiel 2

Eine Mischung von 49,6 g (0,4 Mol) 2-Hydroxymethyl-phenol und 124 g (2 Mol) Äthylmercaptan wird 5 Stunden im Rührautoklaven auf 170°C erhitzt. Die Aufarbeitung wie in Beispiel 1 ergibt 32,2 g (96% der Theorie) 2-Äthylthiomethyl-phenol.

Beispiel 3

Eine Mischung aus 24,8 g 2-Hydroxymethylphenol, 124 g Äthylmercaptan und 40 g Methanol wird im Rührautoklaven 5 Stunden auf 140°C erhitzt, abgekühlt und destillativ aufgearbeitet. Ausbeute 31,3 g (93% der Theorie) 2-Äthylthiomethyl-phenol.

Beispiel 4

Eine Mischung aus 49,6 g (0,4 Mol) 2-Hydroxymethyl-phenol, 24,8 g (0,4 Mol) Äthylmercaptan und 90 g Diisopropyläther wird 5 Stunden in einem VA-Rührautoklaven auf 170°C erhitzt. Nach Abkühlen werden zunächst das Reaktionswasser und das Lösungsmittel bei Normaldruck und dann das 2-Äthylthiomethylphenol bei $<2{,}6 \cdot 10^{-3}$ Bar ($<2$ Torr) abdestilliert. Ausbeute: 60,5 g 2-Äthylthiomethylphenol $\triangleq$90% der Theorie.

Beispiel 5

Eine Mischung aus 49,6 g (0,4 Mol) 2-Hydroxymethyl-phenol, 180 g Diisopropyläther und 29,8 g (0,48 Mol) Äthylmercaptan wird in einem VA-Autoklaven 5 Stunden unter Eigendruck auf 170° erhitzt, abgekühlt und wie in Beispiel 1 aufgearbeitet. Ausbeute an 2-Äthylthiomethyl-phenol: 95% der Theorie, bezogen auf 2-Hydroxymethylphenol.

Beispiel 6

Ein Gemisch aus 450 g Phenol, 0,8 g Zinkacetat und 24 g Paraformaldehyd wird 6 Stunden auf 70° in einem Kolben mit Rührer und Rückflusskühler erwärmt. Dann werden 49,6 g Äthylmercaptan zum Reaktionsgemisch gegeben, und die Reaktionstemperatur im Kolben wird ständig so gesteigert, dass immer ein schwacher Rückfluss vorhanden ist. Nach ca. 12 Stunden, wenn eine Endtemperatur von 120° erreicht ist, wird die Reaktion durch Abkühlen abgebrochen und das Reaktionsgemisch destillativ aufgearbeitet. Das Phenol kann bei der nächsten Reaktion wieder verwendet werden. Die Ausbeute an isoliertem 2-Äthylthiomethyl-phenol beträgt 70% der Theorie, bezogen auf Paraformaldehyd, und ca. 95%, bezogen auf intermediär gebildetes 2-Hydroxymethyl-phenol.

Beispiel 7

Ein Gemisch aus 49,6 g (0,4 Mol) 2-Hydroxymethyl-phenol, 24,8 g (0,4 Mol) Äthylmercaptan und 200 ml o-Dichlorbenzol wird in einem Kolben mit Rührer und Rückflusskühler unter Normaldruck und von Raumtemperatur ausgehend so erhitzt, dass immer ein mässiger Rückfluss besteht und eine Endtemperatur im Kolben von ca. 125° nicht überschritten wird. Insgesamt wird ca. 20 Stunden unter Rückfluss erhitzt. Danach ergibt die destillative Aufarbeitung des Reaktionsgemisches 2-Äthylthiomethyl-phenol in einer Ausbeute von 87% der Theorie. Das abdestillierte o-Dichlorbenzol kann beim nächsten Ansatz wiederverwendet werden.

Beispiel 8

Ein Gemisch aus 62 g (0,5 Mol) 4-Hydroxymethyl-phenol, 37 g (0,6 Mol) Äthylmercaptan und 230 g Phenol wird in einem Kolben mit Rührer und Rückflusskühler unter Normaldruck zunächst innerhalb von ca. 15 Minuten auf ca. 85° und dann weiter so erhitzt, dass stets ein mässiger Rückfluss besteht und schliesslich eine Endtemperatur von ca. 130° im Kolben erreicht ist. Nach einer Reaktionszeit von 18 Stunden bei 85–130° wird abgekühlt und destillativ aufgearbeitet. Man erhält 4-Äthylthiomethyl-phenol in einer Ausbeute von 65% der Theorie.

Beispiele 9 bis 18

49,6 g (0,4 Mol) 2-Hydroxymethyl-phenol werden mit 24,8 g (0,4 Mol) Äthylmercaptan in 200 ml Lösungs- oder Verdünnungsmittel 5 Stunden bei 170° unter Eigendruck in einem VA-Rührautoklaven umgesetzt. Je nach Lösungs- oder Verdünnungsmittel ergibt die destillative Aufarbeitung folgende Ausbeuten an 2-Äthylthiomethyl-phenol (Tabelle 1):

Tabelle 1:

| Beispiel Nr. | Lösungs- oder Verdünnungsmittel | 2-Äthylthiomethyl-phenol Ausbeute, % der Theorie |
|---|---|---|
| 9 | Cyclohexan | 91 |
| 10 | Toluol | 95 |
| 11 | o-Xylol | 91 |
| 12 | Chlorbenzol | 87 |
| 13 | Isopropanol | 90 |
| 14 | Glycolmonomethyläther (Methylglycol) | 95 |
| 15 | Pyridin | 91 |
| 16 | Acetonitrol | 89 |
| 17 | Dimethylformamid | 86 |
| 18 | Wasser | 73*) |

*) Das Reaktionsgemisch besteht aus einer wässrigen Phase und 63 g einer organischen Phase. Die Destillation der organischen Phase ergibt diese Ausbeute

Beispiele 19 bis 24

49,6 g (0,4 Mol) 2-Hydroxymethyl-phenol werden mit 0,4 Mol eines Mercaptans (s. Tabelle 2) in Gegenwart von 200 ml Diisopropyläther in einem VA-Autoklaven 5 Stunden unter Eigendruck auf 170° erhitzt. Nach Abkühlen auf Raumtemperatur wird nach gängigen Arbeitsmethoden aufgearbeitet. Die je nach eingesetztem Mercaptan erhaltenen 2-Alkyl- bzw. 2-Arylthiomethyl-phenole und ihre Ausbeuten sind in Tab. 2 zusammengestellt:

Tabelle 2:

| Beispiel Nr. | Mercaptan | 2-Alkyl- bzw. 2-Arylthiomethyl-phenol Art | % d.Th. |
|---|---|---|---|
| 19 | Methylmercaptan | 2-Methylthiomethyl-phenol | 93 |
| 20 | Isopropylmercaptan | 2-Isopropylthiomethyl-phenol | 88 |
| 21 | n-Butylmercaptan | 2-n-Butylthiomethyl-phenol | 84 |
| 22 | Benzylmercaptan | 2-Benzylthiomethyl-phenol | 90 |
| 23 | Thiophenol | 2-Phenylthiomethyl-phenol | 91 |
| 24 | 4-Chlor-thiophenol | 2-(4-Chlor-phenyl)-thiomethyl-phenol | 70*) |

*) 10 Stunden unter Eigendruck auf 170 °C erhitzt.

Beispiele 25 bis 28

0,4 Mol Hydroxymethylphenol werden in 200 ml Diisopropyläther mit 0,4 Mol Äthylmercaptan pro Mol Hydroxymethylgruppe 5 Stunden lang bei 170° im Rührautoklaven umgesetzt. Nach Abkühlen auf Raumtemperatur wird nach gängigen Arbeitsmethoden aufgearbeitet. Die je nach eingesetztem Hydroxymethylphenol erhaltenen Äthylthiomethylphenole und ihre Ausbeuten sind in Tabelle 3 zusammengestellt.

Tabelle 3:

| Beispiel Nr. | Hydroxymethylphenol | Äthylthiomethylphenol Art | % d.Th. |
|---|---|---|---|
| 25 | 4-Methyl-2-hydroxymethyl-phenol | 4-Methyl-2-äthylthiomethyl-phenol | 91 |
| 26 | 4-Chlor-6-methyl-2-hydroxy-methyl-phenol | 4-Chlor-6-methyl-2-äthylthio-methyl-phenol | 95 |
| 27 | 4,6-Dichlor-2-hydroxymethyl-phenol | 4,6-Dichlor-2-äthylthiomethyl-phenol | 70 |
| 28 | 4-Methyl-2,6-bis(hydroxymethyl)-phenol | 4-Methyl-2,6-bis(äthylthio-methyl)-phenol | 94 |

**Patentanspruch**

Verfahren zur Herstellung von Alkyl- bzw. Arylthiomethylphenolen der Formel

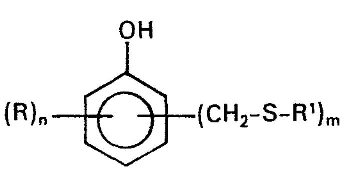

I

in der

m für 1, 2 oder 3 steht,

n für die Zahl steht, die sich aus der Differenz (5-m) ergibt, und

R entweder einzeln und unabhängig voneinander für Wasserstoff, Alkyl, Cycloalkyl, Aryl, Aralkyl, Alkoxy, Cycloalkoxy, Aryloxy, Halogen, Nitro oder für einen an den Phenylkern ankondensierten Benzolring oder Cycloalkanring steht und

$R^1$ für gegebenenfalls substituiertes Alkyl mit 1 bis 12 C-Atomen oder für gegebenenfalls substituiertes Phenyl oder gegebenenfalls substituiertes Aralkyl steht,

dadurch gekennzeichnet, dass man Hydroxymethylphenole der Formel

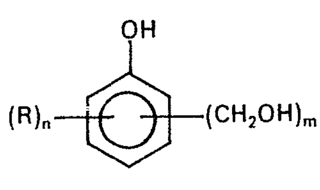

II

mit Mercaptanen oder Thiophenolen der Formel

$R^1$-S-H III

bei Temperaturen von 20 bis 200°, gegebenenfalls in Anwesenheit eines Verdünnungsmittels, umsetzt.

**Claim**

A process for the preparation of alkylthiomethylphenols or arylthiomethylphenols of the formula

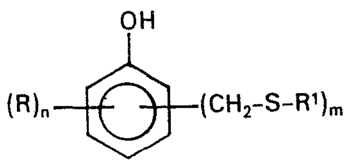

I

in which

m represents 1, 2 or 3,

n represents the number resulting from the difference (5–m) and

each R, independently of any other R, represents hydrogen, alkyl, cycloalkyl, aryl, aralkyl, alkoxy, cycloalkoxy, aryloxy, halogen or nitro, or the R's represent a benzene ring or cycloalkane ring fused to the phenyl ring, and

$R^1$ represents optionally substituted alkyl with 1–12 C atoms or represents optionally substituted phenyl or optionally substituted aralkyl,

characterised in that hydroxymethylphenols of the formula

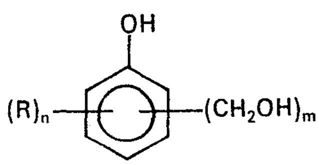

II

are reacted with mercaptans or thiophenols of the formula

$R^1$–S–H (III),

at temperatures of from 20° to 200°C, if appropriate in the presence of a diluent.

**Revendication**

Procédé pour la préparation d'alkyl- ou arylthiométhylphénols de formule

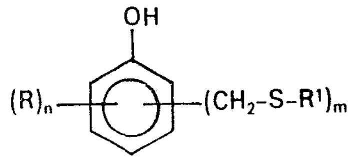

I

dans laquelle

m est égal à 1, 2 ou 3

n est un nombre égal à la différence de (5–m), et

les restes R représentent chacun indépendamment des autres l'hydrogènes ou un groupe alkyle, cycloalkyle, aryle, arylalkyle, alcoxy, cycloalcoxy, aryloxy, halogène, nitro, ou bien représentent un noyau benzénique ou cycloalcane condensé sur le noyau phényle, et

$R^1$ représente un reste alkyle en $C_1$–$C_{12}$ éventuellement substitué ou un [groupe] phényle éventuellement substitué ou un [groupe] arylalkyle éventuellement substitué,

caractérisé en ce que l'on fait réagir des hydroxyméthylphénols de formule

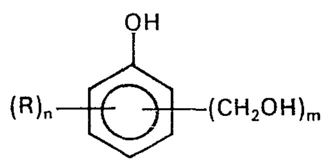

II

avec des mercaptans ou des thiophénols de formule

$R^1$–S–H III

à des températures de 20 à 200°, éventuellement en présence d'un agent diluant.